(19) Europäisches Patentamt
European Patent Office
Office européen des brevets

(11) Publication number: **0 142 571**
**A1**

(12) # EUROPEAN PATENT APPLICATION

(21) Application number: 83111441.8

(22) Date of filing: 15.11.83

(51) Int. Cl.[4]: C 07 C 51/10
C 07 C 59/347

(43) Date of publication of application:
29.05.85 Bulletin 85/22

(84) Designated Contracting States:
AT BE CH DE FR GB IT LI LU NL SE

(71) Applicant: ETHYL CORPORATION
Ethyl Tower 451 Florida Boulevard
Baton Rouge Louisiana 70801(US)

(72) Inventor: Wolfram, Joachim Wilhelm
1224 Bullrush Drive
Baton Rouge Louisiana 70810(US)

(74) Representative: Berg, Wilhelm, Dr. et al,
Patentanwälte Dr. Berg Dipl.-Ing. Stapf Dipl.-Ing.
Schwabe Dr. Dr. Sandmair Postfach 86 02 45
Stuntzstrasse 16
D-8000 München 86(DE)

(54) **Preparation of bis-alpha-keto-carboxylic acids.**

(57) A process for preparing bis-α-keto-carboxylic acids by reacting a dihaloalkane in a liquid solvent medium with carbon monoxide at elevated temperature and pressure in the presence of a catalytic amount of a metal carbonyl compound and an alkali or an alkaline earth metal inorganic base.

EP 0 142 571 A1

Croydon Printing Company Ltd.

Case 5050

# PREPARATION OF BIS-α-KETO-CARBOXYLIC ACIDS

The present invention relates to a process for the biscarbonylation of a functionalized dihaloalkane to form a bis-α-keto-carboxylic acid as the predominant product.

The practical value of such acids and their derivatives is that they can be used in the synthesis of pharmaceuticals, specialty chemicals, amino acids and for preparing polymers.

The preparation of α-keto-carboxylic acids and their derivatives has been the subject of a large number of investigations. According to Rodd, The Chemistry of Carbon Compounds (1952 edition), Vol. 1, pages 227-229, the following methods of preparation are available:

gentle oxidation of α-hydroxyacids containing a secondary hydroxyl group, or by the enzymatic deamination of α-amino-acids;
hydrolysis of an acyl cyanide;
hydrolysis of α-oximino-esters;
from glycidic acid esters on treatment with benzene saturated with boron trifluoride;

from α, β-dibromocarboxylic acids by forming a piperidine addition compound followed by hydrolysis;
from α-keto-acetals by ultraviolet irradiation in the presence of N-bromosuccinimide;
from α-bromomethylketones by boiling with selenium dioxide in absolute methanol or ethanol;
from carboxylic acid esters by oxidation with selenium dioxide;
permanganate oxidation of vinyl ketones;
from carboxylic acid esters by condensation with oxalic ester followed by decarboxylation;
from aldehydes via 5-alkylidene-2-thio-oxazolid-4-ones or by reaction with methyl methoxyacetate;
hydrolysis of azlactones or acetamido-acrylic acids;
hydrolysis of the reaction product of Grignard reagents on diethyl-oxamic ester;
oxidation of α-hydroxyacid esters containing two β-hydrogen atoms by N-bromosuccinimide in carbon tetrachloride to β-bromo-α-keto-acid esters; and

by the action of alkali on the dimethanesulphonates and ditoluene-p-sulphonates of α,β-dihydroxy-carboxylic acids.

It has now been found that bis-α-keto-carboxylic acids of the general formula:

$$HO-\overset{O}{\overset{\|}{C}}\overset{O}{\overset{\|}{C}}-\overset{R}{\overset{|}{C}}H(CHR)_n-CH_2\overset{O}{\overset{\|}{C}}\overset{O}{\overset{\|}{C}}-OH$$

in which R represents hydrogen or a monovalent alkyl radical which can be either straight chain or branched containing from 1 to 40 carbon atoms and n is 5 to 40, can be prepared by carbonylating a dihaloalkane of the general formula:

$$X\overset{R}{\overset{|}{C}}H(CHR)_nCH_2X$$

wherein R and n are as defined above and X is the same halogen selected from either chlorine, bromine or iodine, in a liquid solvent medium, with carbon monoxide at a pressure of from 300 to 3000 psig in the presence of a catalytic amount of a metal carbonyl compound and an alkali or an alkaline earth metal inorganic base.

The terminal dihaloalkane reactants suitable for use in the present process are well known in the art as are methods for their preparation, and as defined above, are of the general formula:

$$X\overset{\overset{R}{|}}{C}H(CHR)_nCH_2X$$

wherein R, n and X are as defined above. A few exemplary materials of this type include:

1,7-dibromoheptane,

1,8-dibromooctane,

1,9-dibromononane,

1,10-dibromodecane,

1,11-dibromoundecane,

1,12-dibromododecane,

1,13-dibromotridecane,

1,14-dibromotetradecane,

1,15-dibromopentadecane,

1,16-dibromohexadecane,

1,17-dibromoheptadecane,

1,18-dibromooctadecane,

1,19-dibromononadecane,

1,20-dibromoeicosane,

1,21-dibromoheneicosane,

1,22-dibromodocosane,

1,23-dibromotricosane,
1,24-dibromotetracosane,
1,25-dibromopentacosane,
1,30-dibromotriacontane,
1,40-dibromotetracontane,
1,7-dichloroheptane,
1,8-dichlorooctane,
1,9-dichlorononane,
1,10-dichlorodecane,
1,11-dichloroundecane,
1,12-dichlorododecane,
1,13-dichlorotridecane,
1,14-dichlorotetradecane,
1,15-dichloropentadecane,
1,16-dichlorohexadecane,
1,17-dichloroheptadecane,
1,18-dichlorooctadecane,
1,19-dichlorononadecane,
1,20-dichloroeicosane,
1,21-dichloroheneicosane,
1,22-dichlorodocosane,
1,23-dichlorotricosane,
1,24-dichlorotetracosane,
1,25-dichloropentacosane,
1,30-dichlorotriacontane,
1,40-dichlorotetracontane,

1,7-diiodoheptane,
1,8-diiodooctane,
1,9-diiodononane,
1,10-diiododecane,
1,11-diiodoundecane,
1,12-diiodododecane,
1,13-diiodotridecane,
1,14-diiodotetradecane,
1,15-diiodopentadecane,
1,16-diiodohexadecane,
1,17-diiodoheptadecane,
1,18-diiodooctadecane,
1,19-diiodononadecane,
1,20-diiodoeicosane,
1,21-diiodoheneicosane,
1,22-diiododocosane,
1,23-diiodotricosane,
1,24-diiodotetracosane,
1,25-diiodopentacosane,
1,30-diiodotriacontane, and
1,40-diiodotetracontane.

Products which can be made by the process of the presentinvention include, by way of example,

2,10-dioxo-undecane-1,11-dioic acid,
2,11-dioxo-dodecane-1,12-dioic acid,
2,12-dioxo-tridecane-1,13-dioic acid,

2,13-dioxo-tetradecane-1,14-dioic acid,
2,14-dioxo-pentadecane-1,15-dioic acid,
2,15-dioxo-hexadecane-1,16-dioic acid,
2,16-dioxo-heptadecane-1,17-dioic acid,
2,17-dioxo-octadecane-1,18-dioic acid,
2,18-dioxo-nonadecane-1,19-dioic acid,
2,19-dioxo-eicosane-1,20-dioic acid,
2,20-dioxo-heneicosane-1,21-dioic acid,
2,21-dioxo-docosane-1,22-dioic acid,
2,22-dioxo-tricosane-1,23-dioic acid,
2,23-dioxo-tetracosane-1,24-dioic acid,
2,24-dioxo-pentacosane-1,25-dioic acid,
2,25-dioxo-hexacosane-1,26-dioic acid,
2,26-dioxo-heptacosane-1,27-dioic acid,
2,27-dioxo-octacosane-1,28-dioic acid,
2,28-dioxo-nonacosane-1,29-dioic acid,
2,33-dioxo-tetratriacontane-1,34-dioic acid,
and
2,43-dioxo-tetratetracontane-1,44-dioic acid.

The reaction is carried out in the presence of a mixture of water and alcohol as a reaction medium. Preferably, the alcohols employed for the reaction may be straight-chain, branched or cyclic, and preferably contain up to 6 carbon atoms. Methanol, ethanol, propanol, isopropanol, n-butanol, isobutanol, tert-butanol, and tert-amyl alcohol may be mentioned as examples.

Cyclic ethers, such as tetrahydrofuran, also may be used. A particularly preferred solvent alcohol is tert-butanol. Mixtures containing 10% to 90% by weight of water and 90% to 10% by weight of alcohol generally are used. Preferred mixtures contain 30% to 80% by weight water and 70% to 20% by weight alcohol.

The reaction takes place in the presence of a basic substance suitably an alkali or an alkaline earth metal hydroxide employing a metal carbonyl compound. During the reaction, the dihaloalkane undergoes reaction with the carbon monoxide and basic substance whereby the desired bis-α-keto-carboxylic acid is formed.

Specific examples of suitable basic agents which can be used in the practice of the process include: LiOH, NaOH, KOH, RbOH, $Ca(OH)_2$, $Ba(OH)_2$ and $Mg(OH)_2$. LiOH and $Ca(OH)_2$ are particularly preferred.

The amount of basic agent used can vary within wide limits. In general, the molar ratio of the alkali or alkaline earth metal base to dihaloalkane reactant is preferably 10:1 to 1:1.

In the process described herein, it is preferred to use metal carbonyl compounds as carbonylation catalysts. These catalysts include particularly metal

carbonyls such as iron pentacarbonyl, dicobalt-octacarbonyl and nickel-tetracarbonyl, or their salts such as, for example, the calcium, potassium or sodium salts thereof. Dicobalt-octacarbonyl is very particularly suited. These catalysts can be added to the medium in the solid state or in the form of solutions in the solvent used for the carbonylation reaction. The molar percentage of the metal carbonyl compound to the dihaloalkane reactant is preferably from 0.1 to 25%.

The concentration of the dihaloalkane reactant used in the reaction solvent is not critical and can vary within wide limits. Thus, it can be between 1 and 30% by weight, based on the weight of the solvent, however, it is possible to go outside of these limits without disadvantage.

The present process is advantageously carried out by bringing the mixture consisting of the dihaloalkane reactant,the metal carbonyl catalyst and the alkali or alkaline earth metal base, suspended in the mixture of water and alcohol, into contact, under nitrogen, in a suitable pressure-resistant reactor equipped with a stirrer, with a large excess of carbon monoxide (amount greater than 2 moles of carbon monoxide per mole of the starting dihaloalkane reactant) introduced at the desired pressure and temperature, in accordance with techniques suitable for bringing about the reaction between a liquid phase and a gas phase.

The carbonylation reaction is carried out at a temperaturein the range of from about 30°C. to 150°C., preferably from 50°C. to 100°C., over a period of time of from 3 to 60 hours, typically 3 to 20 hours.

In general, the reaction takes place at elevated carbon monoxide pressures which may range from 300 psig to 3000 psig. Preferably, the reaction takes place at a pressure in the range of 500 psig to 1000 psig. The carbon monoxide may contain or be mixed with an inert gas, such as nitrogen.

On completion of the reaction, the product mixture is filtered, resulting in the alkali or alkaline earth metal basic reagent being separated from the liquid reaction components as the main solid component. The desired bis-α-keto-carboxylic acid product is easily separated from the resultant reaction mixture by such means as distillation, extraction, crystalliza- tion or the like.

The following examples illustrate the invention.

<u>Example 1</u>

Into a 300 mL autoclave were charged 10 g (38.76 mmoles) of 1,7 dibromoheptane and 70 mLs of t-BuOH. Next, 0.66 g (1.94 mmoles) of $Co_2(CO)_8$ were added under CO, and then a mixture of 11.47 g (155 mmoles) of lime and 30 mLs of $H_2O$ were added. After 860 psi CO

was charged to the autoclave, the reaction mixture was heated to 90°C over a period of time of approximately 1 hour and held at that temperature for 7 hours. After suction filtration, the solid was rinsed once with a 20 mL portion of a 50:50 t-butanol/water solution and then acidified with 150 mLs of HCl solution containing ∿350 mmoles of HCl. To ascertain the yield, 17.5% of the crude solid was acidified with 100 mLs of HCl solution containing 70 mmoles of HCl. The free acid was extracted with diethyl ether (2 x 50 mLs) to give 1.78 g of 2,10-dioxo-undecane-1,11-dioic acid which was 80% pure by VPC analysis of the trimethyl silyl (TMS) derivative. Thus, the total yield of product was 85.8%.

Example 2

Into a 300 mL autoclave were charged 16.49 g (54.97 mmoles) of 1,10 dibromodecane and 70 mLs of t-BuOH. Next, 0.94 g (2.75 mmoles) of $Co_2(CO)_8$ were added under CO, and then a mixture of 16.27 g (220 mmoles) of lime and 30 mLs of $H_2O$ were added. After 860 psi CO was charged to the autoclave, the reaction mixture was heated to 90°C over a period of time of approximately 1 hour and held at that temperature for 7 hours. After suction filtration, the solid was rinsed once with a 20 mL portion of a 50:50 t-butanol/water solution and then acidified with 150 mLs of HCl solution containing ∿350 mmoles of HCl. To ascertain the yield,

9.6% of the crude solid was acidified with 100 mLs of HCl solution containing 50 mmoles of HCl. The free acid was extracted with diethyl ether (2 x 50 mLs) to give 1.45 g of 2,13-dioxo-tetradecane-1,14-dioic acid which was 80% pure by VPC analysis of the trimethyl silyl (TMS) derivative. The total yield of product was 71%.

CLAIMS:

1. A process for the preparation of bis-α-keto-carboxylic acids which comprises reacting a dihaloalkane in a liquid solvent medium with carbon monoxide at elevated temperature and pressure in the presence of a catalytic amount of a metal carbonyl compound and an alkali or an alkaline earth metal inorganic base.

2. A process as claimed in Claim 1 in which the bis-α-keto-carboxylic acids of the general formula

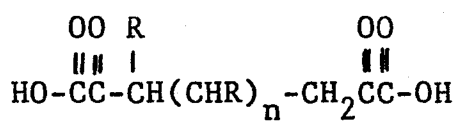

wherein R represents hydrogen or a monovalent alkyl radical which can be either straight chain or branched containing from 1-40 carbon atoms and n is 5 to 40, and which comprises reacting a dihaloalkane of the general formula

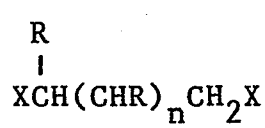

wherein R and n are as defined above and X is the same halogen selected from either chlorine, bromine or iodine, in a liquid solvent medium, with carbon monoxide

at elevated temperature and pressure in the presence of a catalytic amount of a metal carbonyl compound and an alkali or an alkaline earth metal inorganic base.

3. The process as claimed in Claim 2 in which the carbon monoxide pressure is from 300 to 3000 psig.

4. The process as claimed in Claim 2 in which the reaction is carried out at a temperature of from 30°C to 150°C.

5. The process as claimed in Claim 2 in which the inorganic base is selected from LiOH, NaOH, KOH, RbOH, $Ca(OH)_2$, $Ba(OH)_2$ or $Mg(OH)_2$.

6. The process as claimed in Claim 2 in which the metal carbonyl catalyst compound is iron pentacarbonyl, dicobalt-octacarbonyl, or nickel-tetracarbonyl.

7. The process as claimed in Claim 6 in which the metal carbonyl is dicobalt-octacarbonyl.

8. The process as claimed in Claim 2 in which the liquid solvent medium is a mixture of water and alcohol.

9. The process as claimed in Claim 8 in which the alcohol is tert-butanol.

10. The process as claimed in Claim 8 in which the alcohol is isopropanol.

European Patent Office

# EUROPEAN SEARCH REPORT

0142571
Application number

EP 83 11 1441

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (Int. Cl. 3) |
| A | US-A-2 776 926 (SHARPE et al.)<br>* Claims *<br>----- | 1 | C 07 C 51/10<br>C 07 C 59/347 |
| | | | TECHNICAL FIELDS SEARCHED (Int. Cl. 3) |
| | | | C 07 C 51/00<br>C 07 C 59/00 |

The present search report has been drawn up for all claims

| Place of search | Date of completion of the search | Examiner |
|---|---|---|
| THE HAGUE | 10-07-1984 | KLAG M.J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document